(19) Europäisches Patentamt
European Patent Office
Office européen des brevets

(11) Publication number: 0 285 302 A1

# (12) EUROPEAN PATENT APPLICATION

(21) Application number: 88302483.8

(51) Int. Cl.4: C07C 119/02 , A61K 31/275

(22) Date of filing: 22.03.88

(30) Priority: 30.03.87 US 32289

(43) Date of publication of application: 05.10.88 Bulletin 88/40

(84) Designated Contracting States: DE ES FR GB IT

(71) Applicant: HARBOR BRANCH OCEANOGRAPHIC INSTITUTION, INC.
5600 Old Dixie Highway
Fort Pierce, FL 33450(US)

(72) Inventor: Wright, Amy E.
Anchor Way,
Ft.Pierce, FL. 33450(US)
Inventor: McCarthy, Peter.
1771 Gulfstream Avenue,
Ft. Pierce, FL. 33449(GB)
Inventor: Cross, Sue Shipley
1915 Jacaranda Drive,
Ft. Pierce, FL. 33449(US)
Inventor: Rake, James B.
39 Westchester Drive, Clifton Park,
NY 12065(US)
Inventor: McConnell, Oliver J.
156 22nd Avenue, Vero Beach,
FL. 32862(US)

(74) Representative: Marlow, Nicholas Simon et al
Reddie & Grose 16, Theobalds Road
London WC1X 8PL(GB)

## (54) Sesquiterpenoid isocyanide composition and its methods of use.

(57) This invention relates to a novel composition which is useful as an antitumor, antiviral and antifungal composition, a process of producing the composition and a method for inhibiting tumors, viruses, and fungi utilizing the composition. More particularly, the novel composition is an organic sesquiterpenoid isocyanide composition which is derived from marine sponge Bubaris sp., and has the general formula:

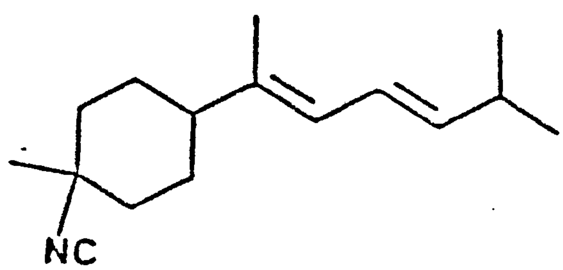

EP 0 285 302 A1

# SESQUITERPENOID ISOCYANIDE COMPOSITION AND ITS METHODS OF USE

This invention relates to a new organic composition which has useful antitumor. antiviral and antifungal activity.

Various tumor related diseases inflict man. Considerable research has been devoted to oncology and antitumor measures. Tumors are common in a variety of mammals and the prevention. control of the growth and regression of tumors in mammals is important to man. The term tumor refers to abnormal masses of new tissue growth which is discordant with the economy of the tissue of origin or of the host's body as a whole.

Tumors inflict mammals and man with a variety of disorders and conditions including various forms of cancer and resultant cancerous cachexia. Cancerous cachexia refers to the symptomatic discomfort that accompanies the infliction of a mammal with a tumor. These symptoms include weakened condition of the inflicted mammals as evidenced by, for example, weight loss. The seriousness of cancer is well known. e.g.. cancer is second only to heart and vascular diseases as a cause of death in man.

Viral diseases inflict man, plants, insects, and animals. The prevention and control of viral diseases have important health and economic implications.

Viral diseases contribute to inflictions in humans including common colds, herpes and cancer and the importance of their control is obvious. Also important is control of viral diseases in animals for economic reasons as well as the ability of such animals to become virus reservoirs or carriers which facilitate the spreading of viral diseases to humans. Viral plant diseases have been known to have a disruptive effect on the cultivation of fruit trees, tobacco, and various vegetables. Insect viral diseases are also of interest because of the insects' ability to transfer viral diseases to humans.

Prevention of the growth of fungus and the infections and maladies caused by it to mammals and plants is also of importance to man. The presence of fungus may cause various diseases and infections in man including mycotic disease, e.g., pulmonary candidiasis and pulmonary blastomycosis. Certain yeastlike organisms, e.g., Cryptococcus neoformans, may cause serious infections of the central nervous system. More commonly known fungal infections in humans and mammals include ringworm, which are fungus infections of hair and nail areas, as well as resistant infections of the skin. Many other fungal infections inflict humans and mammals in the areas of skin, mucous membranes, intestinal tract, vaginal area and lungs.

Plants are also attacked by various fungi. Damage caused by fungus infection to agriculture amounts to billions of dollars annually. Various inorganic and organic fungistats and fungicides have been tried but with limited success. It is of course important for the fungistat or fungicide to kill the fungi but not the plant and to leave no toxic residue on the food of the plant. Various methods have been utilized to combat fungus infection in agriculture including foliage fungicide by which method plants are coated with a preventive weather-resistant fungicide. Seed treatment and soil treatment are methods which require fungicides which are safe for seeds and resist degradation by soil and soil microorganisms. Chemotherapeutants are fungicides which permeate the plant to protect new growth or eliminate infections which have already occurred within the plant. Agricultural fungistats and fungicides and their application must also meet very stringent requirements and regulations, which have been promulgated, for example, in the United States.

Considerable research and resources have been devoted to oncology and antitumor measures including chemotherapy; antiviral measures; and combating fungal infections in both mammals and plants. While various antitumor, antiviral or antifungal agents and methods have been developed which aid in inhibiting tumors, viruses and the spread of fungus, respectively, additional methods and chemical agents are needed.

A potential source for antitumor, antiviral, and antifungal compositions is marine plant and animal life and of particular interest are marine sponges. It has now been found that an organic composition derived from extracts of the sponge Bubaris sp. possess useful antitumor, antiviral and antifungal activity.

Some compounds of interest have been previously isolated from marine sponges. In particular sesquiterpenoid compounds have been reported by H. Nakamura, J. Kobayashi, Y. Ohizumi. and Y. Hirata Tetrahedron Letters, Vol. 25, No. 47, Pp. 5401-5404 (1984). This paper discloses the isolation from the Okinawan sea sponge Theonella cf. swinhuei of "theonellin" compositions of the formulae:

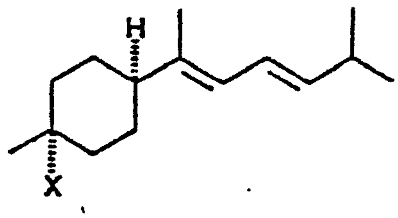

2: X=NCS

3: X=NHCHO

4: X=$NHCH_3$

5: X=$\overset{+}{N}(CH_3)_3 \cdot OH^-$

This paper states at page 5403 that the preparation of derivative compositions may be possible but that an isocyanide derivative has not yet been prepared or isolated. No biological activity has been reported for theonellin compositions.

The invention comprises a composition of the formula:

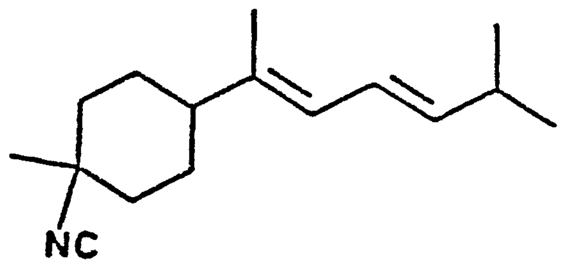

I

In preferred embodiments of the invention, the composition is substantially pure.

As embodied and fully described herein, the invention also comprises an antitumor, antiviral or antifungal composition comprising, as active ingredient, an effective antitumor, antiviral or antifungal amount, respectively, of the composition of the invention and a non-toxic pharmaceutically acceptable carrier or diluent.

As embodied and fully described herein, the invention also comprises a process to produce the composition of the invention. The process comprises the steps of collecting marine sponge Bubaris, sp. contacting the marine sponge with a suitable organic solvent system to obtain an extract; fractionating the extract; and isolating the sesquiterpenoid isocyanide composition of the invention from the fractionated extract.

As embodied and fully described herein, the invention further comprises a method for inhibiting tumors comprising contacting tumor cells with an effective antitumor amount of the composition of the invention.

As embodied and fully described herein, the invention further comprises method for inhibiting viruses comprising contacting viruses with an effective antiviral amount of the composition of the invention.

As embodied and fully described herein, the invention further comprises a method for inhibiting the growth of or killing fungi comprising contacting fungi with an effective antifungal amount of the composition of the invention.

It is to be understood that both the foregoing general and the following detailed description are exemplary and explanatory only and are not intended to be restrictive of the invention as claimed.

## DETAILED DESCRIPTION OF THE PREFERRED EMBODIMENTS OF THE INVENTION

Reference will now be made in detail to present preferred embodiments of the invention, examples of which are illustrated in the following example section.

In accordance with the invention novel compositions are provided to achieve the objects in accordance with the purposes of the invention, as embodied and fully described herein, the invention comprises a sesquiterpenoid isocyanide composition of the general formula I:

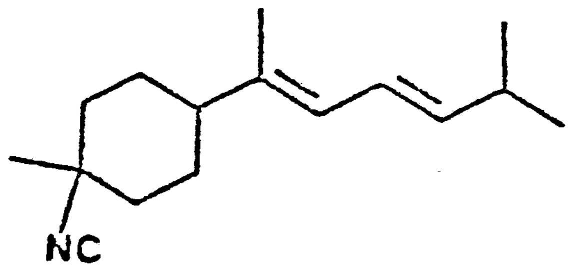

I

In preferred embodiments of the invention, the composition is substantially pure.

In accordance with the invention, an antitumor composition is provided comprising as active ingredient an effective antitumor amount of the composition of the invention and a non-toxic pharmaceutically acceptable carrier or diluent. While effective amounts may vary, as conditions in which an antitumor composition is used vary, a minimal dosage required for activity is generally between 0.01 and 10 micrograms against $10^5$ tumor cells. Useful examples of non-toxic pharmaceutically acceptable carriers or diluents include, but are not limited to, the following: ethanol, dimethyl sulfoxide and glycerol.

In accordance with the invention, a method for inhibiting tumors in a host is provided comprising contacting a tumor with an antitumor amount of the composition of the invention. The composition of the invention is active for inhibiting a diverse range of tumors including, but not limited to P388 mouse leukemia cells, human lung, colon and mammary tumors such as lung carcinoma A-549, ileocecal adenocarcinoma HCT-8, and human breast adenocarcinoma cells MDA-MB-231. The effectiveness of the composition of the invention for inhibiting tumors cells and tumors indicates its usefulness for controlling tumors in hosts including mammals and for treating cancerous cachexia.

In accordance with the invention, an antiviral composition is provided comprising as active ingredient an effective antiviral amount of the composition of the invention and a non-toxic pharmaceutically acceptable carrier or diluent. While effective amounts may vary, as conditions in which an antiviral compositions is used vary, a minimal dosage required for activity is generally between 50 to 100 micrograms against 25 to 80 plaque forming units of virus. The composition of the invention is active for inhibiting or killing a diverse range of viruses including, but not limited to, RNA viruses, vesicular stomatitis (herein "VSV") adeno-, corona-, reo-and influenza viruses and the DNA virus, herpes simplex - I and II (herein "HSV-I" and "HSV-II") adeno-and papova-viruses. Useful examples of non-toxic pharmaceutically acceptable carriers or diluents include, but are not limited to, the following: ethanol, dimethyl sulfoxide and glycerol.

In accordance with the invention, a method for inhibiting viruses in a host is provided comprising contacting viruses with an antiviral amount of the composition of the invention. The effectiveness of the composition of the invention for inhibiting viruses indicates their usefulness for controlling viruses and virus related diseases in hosts including mammals and plants.

In accordance with the invention, an antifungal composition is provided comprising as active ingredient an effective antifungal amount of the composition of the invention and a non-toxic pharmaceutically acceptable carrier or diluent. While effective amounts may vary, as conditions in which an antifungal composition is used vary, a minimal dosage required for activity is generally between 1 and 10 micrograms/ml against $10^3$/ml fungi, such as Candida albicans for example. Useful examples of non-toxic pharmaceutically acceptable carriers or diluents include, but are not limited to, the following: ethanol, dimethyl sulfoxide and glycerol.

In accordance with the invention, a method for inhibiting fungus in a host is provided comprising contacting fungus with an antifungal amount of the composition of the invention. The effectiveness of the composition of the invention for inhibiting fungus indicates its usefulness for controlling fungus and fungus related diseases in hosts including mammals. Further, this composition may be useful as an agricultural fungicide.

In accordance with the invention, a process is provided to produce the compositions of the invention. The process comprises the steps of collecting samples of the marine sponge Bubaris, sp. contacting the marine sponge with a suitable organic solvent system to obtain an extract; partitioning said extract by chromatography to obtain a number of fractions; and isolating the composition of the invention from the fractionated extract.

In preferred embodiments of the invention the suitable organic solvent system is selected from the

group of solvents consisting of ethyl acetate, heptane, hexanes, isooctane, acetone, methanol, benzene, toluene, diethyl ether, t-butyl-methyl ether, methylene chloride, chloroform, ethanol, isopropanol, 1,2-dichloroethane, dichloromethane, and mixtures thereof. A particularly preferred extracting solvent is ethyl acetate.

While those solvents listed above are the presently preferred choices for the solvents useful in accordance with the invention, other suitable solvents may be substituted. A suitable solvent system should be capable of extracting the composition of the invention from other components of the sponge. Different ratios of solvents and any combination may be used in the solvent system of the invention as would be known to those skilled in the art.

Compositions according to the invention are synthesized and/or isolated by various fractionation, and chromatographic techniques from the extracts obtained. Any suitable fractionation and isolation techniques as known to those skilled in the art may be utilized in accordance with the process of the invention. Preferred isolation techniques include various chromatography techniques such as vacuum liquid chromatography or with suitable stationary phases as would be known to those skilled in the art (e.g., Kiesel gel 60-H) eluted with a suitable solvent such as, for example, heptane, methanol, dichloromethane, ethyl acetate, hexanes, isooctane, chloroform, dichloromethane, 1,2-dichloroethane, benzene, toluene, isopropanol, diethyl ether and mixtures thereof. Particularly preferred eluents are heptane, dichloromethane, ethyl acetate, methanol and mixtures thereof.

A more detailed description and explanation of a preferred embodiment of the process of the invention to produce the composition of the invention is provided in the examples section.

It is therefore apparent that the composition of the invention, the process for producing the composition of the invention and the methods for utilizing the composition of the invention to inhibit tumors, viruses and fungus growth fulfill the objects of the invention.

EXAMPLE

The invention will now be illustrated by example. The example is not intended to be limiting of the scope of the present invention. In conjunction with the detailed and general description above, the example provides further understanding of the present invention and outlines a process for producing the composition of the invention.

The following example represents preferred embodiments of the compositions, processes and methods of the invention for satisfying the stated objects of the invention. The starting materials and reagents in the example whose methods of preparation are not indicated are commercially available from sources known to the art such as chemical supply houses.

EXAMPLE 1

Preparation of Sesquiterpenoid Isocyanide

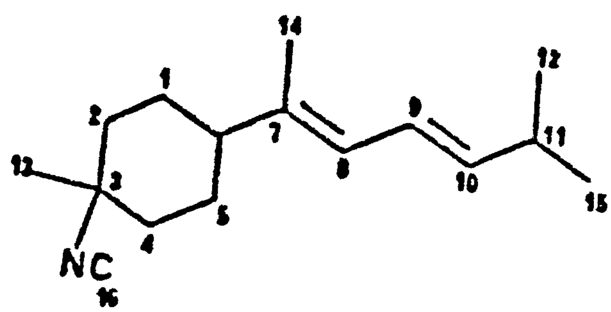

1

The marine sponge Bubaris sp. was collected at a depth of 591 feet in the Bahamas. An extract of 150 gms. of the sponge was prepared by repeated homogenization of the frozen organism with ethyl acetate. 690 milligrams of a crude oil was obtained following filtration and concentration of the extract extraction solvent. The oil was chromatographed by vacuum liquid chromatography on Kiesel gel 60-H (EM Science)

using the eluents outlined below. 94 milligrams of pure 1 is eluted in fraction 6.

| FRACTION | ELUENT | | VOL (ml) | WEIGHT (mg) |
|---|---|---|---|---|
| 1 | 100% heptane | | 100 | 173.9 |
| 2 | 10% $CH_2Cl_2$-90% HEPTANE | | 50 | 33.8 |
| 3 | 20 | 80 | 50 | 9.2 |
| 4 | 30 | 70 | 50 | 7.5 |
| 5 | 40 | 60 | 50 | 64.9 |
| 6 | 50 | 50 | 50 | 93.9 |
| 7 | 60 | 40 | 50 | 42.3 |
| 8 | 70 | 30 | 50 | 7.4 |
| 9 | 80 | 20 | 50 | 19.9 |
| 10 | 100 $CH_2Cl_2$ | | 100 | 22.0 |
| 11 | 100 ETOAC | | 100 | 21.2 |
| 12 | 100 MEOH | | 100 | 97.9 |

Composition 1 is a colorless oil for which no measurable rotation is observed. The spectral characteristics of 1 are listed below. IR: $CCl_4$; 3000-2620, 2105, 1435, 1370, 1120, 960 and 875 $cm^{-1}$

UV: MEOH; $\lambda_{max}$ = 238 nm ($\epsilon$ = 22,500)

MS: 70 EV; m/z = 231, 204, 161, 122, 95, 81, 67

NMR:

PROTON:

1.00 (6H, d J = 6.7; H12abc and H15 abc); 1.42 (3H, bs; H13abc); 1.71 (3H, s H14abc); 1.3-1.4 (4H, m H2ab and H4ab); 1.8-1.9 (4H, m; H1ab and H5ab); 1.98 (1H, m; H6); 2.34 (1H, 6 line pattern; H11); 5.58 (1H, dd J = 15.2, 6.4; H10); 5.79 (1H, dd J = 10.6, 0.7; H8); 6.20 (1H, ddd J = 15.2, 10.6, 1.1; H9)

CARBON:

15.17 (q; C14); 22.47 (2C, q; C12 and C15); 25.01 (q; C13); 25.92 (2C, t; C1 and C5); 31.35 (d; C11); 38.6 (2C, t; C2 and C4); 44.65 (d; C6); 56.4 (bs; C3); 123.3 (d; C9); 123.81 (d; C8); 138.58 (s; C7); 140.63 (d; C10); 152.18 (s; C16)

## ANTITUMOR ACTIVITIES OF THE COMPOSITION OF THE INVENTION

The following assay method was utilized to illustrate the antitumor effectiveness of the sesquiterpenoid composition of the invention.

## P388 MOUSE LEUKEMIA CELL ASSAY

### Maintenance of Cell Line

P388 mouse leukemia cells are grown in Dulbecco MEM medium with 10% horse serum, 4mM glutamine, and 20µg/ml gentamycin (Biologos, Inc.). Cells are incubated in 10% $CO_2$ and subcultured 2 times per week.

### PROCEDURE

1. Add composition to each well of a 24-well plate or tube and allow solvent to evaporate to dryness.
2. Add 2ml (1.2 x $10^5$) cells to each well or tube and mix.
3. Incubate in 10% $CO_2$ at 37°C for 48 hours.

4. Read plates with an inverted microscope, scoring activity from 1+ to 4+ as follows: ND (not detectable), >90%; 1+, 75-90%; 2+, 50-74%; 3+, 25-49%; 4+, <25% of control growth.

Cell counts are performed on each tube and results are reported as percent of control. $IC_{50}$ is the concentration of composition required to inhibit 50% of cell growth.

HUMAN TUMOR CELL LINE ASSAY

Maintenance of Cell Line

HCT-8 human colon tumor cells are grown in RPM1 1640 medium (GIBCO). A-549 human lung carcinoma cells and MDA-MB-231 human breast cancer cells are cultured in Dulbecco medium (Biologos, Inc.). All media are supplemented with 10% fetal bovine serum and contain 50 µg/ml gentamycin. All human tumor cell lines are incubated at 5% $CO_2$ at 37°C and subcultured once a week.

PROCEDURE

1. Seed 1ml cell (5000 HCT-8, 8000 A549, 12000 MDA-MB-231) in each well of a 24-well plate.
2. Incubate in a $CO_2$-incubator for 48 hours.
3. Add composition to each well and incubate for an additional 120 hours.
4. Discard medium and stain with methylene blue (HCT-8) or crystal violet (A-549 and MDA-MB-231).
5. Compare cell density of drug-treated well with that of the control (no drug added) as follows: ND (not detectable), >90%; 1+, 75-90%; 2+, 50-74%; 3+, 25-49%, 4+, <25% of control growth. Positive control - Vinblastine or Vincristine in aqueous solution.

Final Conc. of Vinblastine or Vincristine control (use 2 µl/assay)

| Solution Conc. | Amt added | Final conc. in test |
|---|---|---|
| 5 mg/ml | 2 µl | 5 µg/ml |
| 1 mg/ml | 2 µl | 1 µg/ml |
| 0.1 mg/ml | 2 µl | 0.1 µg/ml |
| 0.05 mg/ml | 2 µl | 0.05 µg/ml |

The results of the above assays are summarized in Table 1.

ANTIVIRAL ACTIVITIES OF THE COMPOSITION OF THE INVENTION

The following assay method was utilized to demonstrate the *in vitro* antiviral effectiveness of compositions of the invention as reported in Table 2.

ANTIVIRAL ASSAY FOR MOUSE CORONAVIRUS A-59

A. CELL CULTURE

1. NCTC clone 1469 is a derivative of mouse liver.
2. ATCC No. CCL 9.1, freeze 2518, passage no. 16, frozen November 1980 at 2.4 X $10^3$.
3. Received January, 1985, and reconstituted March, 1985.
4. Frozen in liquid nitrogen and reconstituted July, 1985.

B. MAINTENANCE OF CELL CULTURE

1. Trypsinization

a. Aseptically remove the medium.
b. Rinse cell sheet with 10 ml of $Ca^{++}$ and $Mg^{++}$ free phosphate buffered saline.
c. Add 4 ml of trypsin-EDTA mixture to a 150 $cm^2$ flask.
d. Leave for one minute or less and then shake flask.
e. Add 10 ml of growth medium and break up cell clumps with pipetting.
f. Count cells.

2. Subcultures for maintenance of cells for assays

a. Seed 150 $cm^2$ tissue culture flasks with 10 x $10^6$ cells in 40 ml growth medium.
b. Subculture 2 times per week.

C. VIRUS

Mouse hepatitus virus strain MHV-A59 classified as a coronavirus.

D. PREPARATION OF PLATES FOR VIRAL ASSAYS

1. Cell Concentration

a. Dilute the cells with growth medium between 5 x $10^5$ and 7.5 x $10^5$ and 7.5 x $10^5$ cells per ml.
b. Seed 24 well trays with 1.0 ml per well.

E. VIRAL ASSAY

1. Dilute drug or extract for test in the appropriate solvent.
2. Add 20 lambda to a 12 mm by 75 mm glass tube for a 16 mm test well.
3. Allow the solvent to evaporate under the laminar flow hood.
4. Dilute the MHV-A59 in Dulbecco's phosphate buffered saline with $Ca^{++}$ and $Mg^{++}$ to the appropriate predetermined dilution for the lot number currently in use. The dilution of virus in a titration which gives a 3+ to 4+ CPE in 24 hours is used in this assay.
5. Remove medium from wells of plates containing NCTC 1469 cells seeded 24 hours earlier.
6. Add 200 lambda of diluted virus to each test well. Add PBS to control wells.
7. Incubate cells and virus for 1 hour at 37°C.
8. Pour off supernatant at end of incubation period.
9. To each glass tube add 10 lambda of dimethyl sulfoxide (DMSO).
10. Add 1 ml of maintenance medium to each glass tube.
11. Pour the contents of the glass tube into the corresponding well of the tissue culture plate.
12. Incubate infected cells at 37°C and read the following day.
13. At twelve hours areas of cell fusion are quite apparent and can be detected both visually and microscopically.
14. At 24 hours the CPE is extensive and on stained plates the difference between activity and none is apparent from visual examination.
15. To stain plates discard medium and to each 16 mm well add 200 lambda of methylene blue stain.
16. Leave the stain on the cell sheet for 30 minutes or more.
17. Pour off the stain and wash plates in tap water until the water is clear.
18. Allow plates to dry.
19. Scoring drug activity

a. Cytotoxicity scoring

100% = complete cell destruction
75% = partial cell destruction

50% = partial cell destruction
25% = partial cell destruction
0% = no cytotoxicity

b. Antiviral activity is scored from 0 to + + +.

+ + + = complete inhibition of cytopathic effects and cell fusion
+ + = partial inhibition
+ = partial inhibition
+/- = partial inhibition
0 = no protection

F. MEDIA

1. Growth medium for NCTC 1469 cell line

Medium NCTC 135 (GIBCO Cat. #320-1350)
10% horse serum
2% 200 mM 1-glutamine
1% nonessential amino acids (NEAA) (100X)
1% sodium pyruvate (110mg/liter) (100X)
gentamicin 10 mg/ml (use 50 µg/ml) or 0.5 ml/100
50 mg/ml (use 50 µg/ml) or 0.1 ml/100

2. Maintenance medium for viral assay

Dulbecco's modified Eagle medium @4500 mg/L glucose
GIBCO Cat. #320-1965
5% fetal bovine serum (FBS)
2% 200 mM 1-glutamine
1% nonessential amino acids (NEAA) (100X)
1% sodium pyruvate (110 mg/liter) (100X)
gentamicin (use 50 µg/ml)

3. Trypsin

GIBCO Cat. #610-5405 trypsin-EDTA (10X)
5.0 gm trypsin (1:250) and 2.0 gm EDTA/L

Prepare 1X solution in Dulbecco's phosphate buffered saline free of $Ca^{++}$ and $Mg^{++}$. Add 1.1 grams glucose per liter.

4. Stain

Methylene Blue-certified
Sigma No. M 9140
50% ethanol:water
5 grams methylene blue/liter

Antiviral Disc Assay for VSV

A. Maintenance of Cell Cultures

1. Virus

a. Vesicular stomatitis virus (VSV) replicate in the CV-1 cell line. CV-1 is a fibroblast-like cell culture derived from primary African green monkey cells.

2. Growth of CV-1 Cells

a. Seed 150cm² tissue culture flasks each with 10 x $10^6$ CV-1 cells in 40 ml of EMEM with 10% FBS (growth medium).

b. Seven days after seeding the flasks cell numbers should be approximately 40-50 x $10^6$ cells. CV-1 cells have a doubling time of 72 hours based on these numbers.

Trypsinization

a. Aseptically remove the medium.

b. Rinse cell sheet with 10 ml of $Ca^{++}$ and $Mg^{++}$ free Dulbecco's phosphate buffered saline or Pucks G saline at least twice.

c. Add 4.0 ml of trypsin -EDTA mixture.

d. Incubate flask at room temperature with occasional rocking until the cells detach from the flask (about 5 min).

e. Shake flask.

f. Add 10 ml EMEM growth medium and break up cell clumps with pipetting.

g. Count cells.

B. Preparation of plates for viral assays

1. Cell Concentration

a. Dilute the cells with EMEM to 4 x $10^5$ cells/ml.

b. Seed 24 well trays with 0.5 ml per well. Cell concentration per well is 2 x $10^5$ cells.

c. Incubation at 37°C with 5% $CO_2$.

d. The wells can be uses over the next several days beginning the day after seeding (preferably 2, 3, or 4).

C. Assay of VSV in CV-1 cells

1. Infection of CV-1 cells in plates with virus

a. Remove medium from wells.

b. Infect well with at least 25 and no more than 80 plaque forming units (PFU) of virus.

c. Incubate infected cells at 37° C for 1.0 hour.

d. Pour off supernatant at end of incubation period.

e. Add 0.5 ml of methylcellulose overlay medium (MCO which is a maintenance medium without phenol red made with 1% 4000 centipose methylcellulose. FBS is used at 5% level).

2. Drug Evaluation

a. For drug evaluation wet filter paper discs (6 mm diameter) with approximately 0.02 ml of marine extract or test compound.

1) Allow solvent to evaporate for 20 to 30 minutes at room temperature.

2) Place discs in the well containing CV-1 cells, virus, and MCO.

b. Incubate tissue culture plates for 48 hours at 37° C.

c. After 48 hours place 0.5 ml NRMCO on each well. NRMCO is a maintenance overlay medium without phenol red containing 0.1 mg neutral red dye per ml and 2% 15 centipoise methylcellulose.

d. Incubate plates at 37° C and read the following day. Antiviral activity should be observed from two parameters. One is actual reduction in the number of plaques and two is the diminution in plaque diameter.

3. Scoring Drug Activity

a. Antiviral activity is scored from 0 to + + +.

+ + + = complete inhibition of plaque formation

+ + = partial inhibition

+ = partial inhibition

+/- = partial inhibition

0 = no protection

b. Cytotoxicity

Wells of 24 well tissue culture plates are 16 mm in diameter. Discs are 6 mm in diameter. Zones of cytotoxicity greater than 6 mm are graded from 8 to 16 using only even numbers.

0 = no visual or microscopic cytotoxicity

16 = Complete cell destruction

8, 10, 12, 14 = partial cytotoxicity

ANTIFUNGAL ACTIVITIES OF THE COMPOSITIONS OF THE INVENTION

The following assay method was utilized to demonstrate the in vitro antifungal effectiveness of compositions of the invention as reported in table 3.

Preparation of inoculum

Candida albicans:

C. albicans(Ca) is grown on Sabouraud dextrose agar to produce single colonies one of which is used to inoculate Sabouraud dextrose broth. The broth is incubated at 37°C with shaking at 200rpm for 18hrs., the resultant culture is frozen with 10% (v/v) glycerol at -80°C and used as the inoculum for the anti-Candida assay.

Assay protocols

1. Disc diffusion assay

C. albicans is inoculated into melted Sabouraud dextrose agar at 45°C to give a cell density of approximately 1000 cells/mL. Plates are prepared with 10mL of the seeded agar in a 10cm x 10cm petri dish. These plates are stored at 4°C until needed for the assay.

Paper discs (6.35mm) are impregnated with the test substance and allowed to dry. They are then placed onto the surface of a test plate prepared as detailed above. Plates are incubated overnight at 37°C after which time the zones of growth inhibition can be read, these are expressed as the diameter of the zone in millimeters.

2. Minimum inhibitory concentration (MIC)

Two-fold dilutions for the drug are prepared in 50µL volumes of Sabouraud dextrose broth using 96-well microtiter plates. An inoculum of C. alibicans is added in a small volume to give a cell density of approximately 1000 cells/mL. Plates are incubated at 37°C overnight. 10µL of Triphenyl tetrazolium chloride (1% w/v) is then added to each well; a further 2 hour incubation results in a deep coloration of the microorganism. The MIC is the lowest concentration of the drug which has completely inhibited growth.

## Table 1

### Antitumor

Mouse: P388: $IC_{50}$ = 0.4 µg/ml (µg/ml)

| Human: Tumor | Cell Line | dose 100 (µg/ml) | 10 | 1 | .1 |
|---|---|---|---|---|---|
| Colon | HCT-8 | 4+ | 3+ | 1+ | 1+ |
| Lung | A-549 | 4+ | 1+ | ND | ND |
| Mammary | MDA-MB-231 | 4+ | 1+ | ND | ND |

## Table 2

### Antiviral

| Virus | Dose: | 200 | 20 | 2 |
|---|---|---|---|---|
| VSV | µg/0.5 ml | 14 +++ | 8 + | 0 +/- |
| A-59 | µg/ml | 75 | 0 ++ | 0 +/- |

## Table 3

### Antifungal

*Candida albicans* MIC=16 µg/ml

The above data reports the *in vitro* activity of the composition of the invention for inhibiting tumors, viruses and fungi. The above results indicate, as would be known to those skilled in the art, that the composition of the invention is useful for inhibiting tumors, viruses and fungi in hosts and the diseases caused thereby.

The composition described herein may have other useful applications such as, for example, analgesic applications or as starting materials for the preparations of other compositions. Therapeutic application of the compositions of the present invention can be accomplished by any suitable therapeutic method and technique as is presently or prospectively known to those skilled in the art. Further, the composition of the invention may have use as a starting material or intermediate for the preparation of other useful compositions.

## Claims

1. A composition according to the formula:

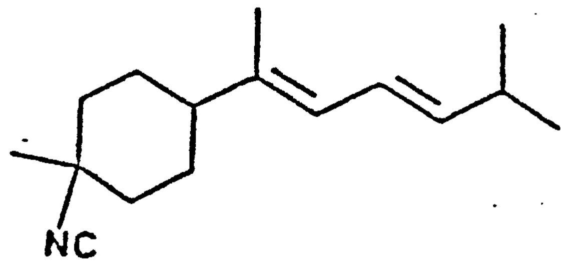

2. A composition according to claim 1, the composition being substantially pure.

3. An antitumor, antiviral or antifungal composition comprising, as active ingredient, an effective antitumor amount of the composition of claim 1 or 2 and a non-toxic pharmaceutically acceptable carrier or diluent.

4. A process to produce a composition according to claim 1 or 2 comprising:
collecting marine sponge genus Bubaris, sp.;
contacting the sponge with a suitable organic solvent system;
obtaining a solvent extract from the sponge;
fractionating the extract; and
isolating a composition according to claim 1 or 2 from the fractionated extract.

5. A process according to claim 4 in which the solvent system is ethyl acetate, heptane, hexanes, isooctane, acetone, methanol, ethanol, isopropanol, toluene, benzene, diethyl ether, t-butyl, methyl ether, chloroform, 1,2-dichloroethane, dichloromethane or any combination thereof.

6. Process according to claim 4 or 5 in which the solvent extract is fractionated and isolated by vacuum liquid chromatography using an eluent consisting of heptane, hexanes, isooctane, chloroform, dichloromethane, 1,2-dichloroethane, benzene, toluene, isopropanol, diethyl ether, methanol or combinations thereof.

7. A method for inhibiting tumors, viruses or fungus growth comprising contacting a tumor, viruses or fungus with an effective antifungal amount of the composition of any of claims 1 to 3.

8. A method according to claim 7 of inhibiting tumors, viruses or fungus growth in a host.

9. A method according to claim 8 for inhibiting tumors, viruses or fungus growth in a mammalian host.

10. A therapeutic method for treating cancerous cachexia caused by the presence of a tumor in a host comprising contacting cells of said tumor with an effective antitumor amount of a composition according to claim 1, 2 or 3.

European Patent Office

# PARTIAL EUROPEAN SEARCH REPORT

which under Rule 45 of the European Patent Convention shall be considered, for the purposes of subsequent proceedings, as the European search report

Application number

EP 88302483.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE JOURNAL OF ORGANIC CHEMISTRY, vol. 51, 1986<br>N.K. GULAVITA et al.: "Nitrogenous Bisabolene Sesquiterpenes from Marine Invertebrates"<br>pages 5136-5139<br>-- | 1,2,4-6 | C 07 C 119/02<br>A 61 K 31/275 |
| D,A | TETRAHEDRON LETTERS, vol. 25, no. 45, 1984, Oxford, New York, Paris, Frankfurt<br>HIDESHI NAKAMURA et al.: "Novel bisabolene-type sesquiterpenoids with a conjugated diene isolated from the Okinawan sea sponge Theonella cf. swinhoei"<br>pages 5401-5404<br>---- | 1,4-6 | |

| TECHNICAL FIELDS SEARCHED (Int. Cl.4) |
|---|
| C 07 C 119/00 |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6

Claims searched incompletely: -

Claims not searched: 7-10

Reason for the limitation of the search:

(method for treatment of the human or animal body by therapy, article 52(4) EPC)

| Place of search | Date of completion of the search | Examiner |
|---|---|---|
| VIENNA | 27-05-1988 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document